# EUROPEAN PATENT SPECIFICATION

(11) **EP 4 701 731 B1**
(45) Date of publication and mention of the grant of the patent: **09.09.2026**
(21) Application number: 24732581.4
(22) Date of filing: 07.06.2024
(51) Int. Cl.: A61N 5/10, H05H 7/04, G21K 1/093

(54) **BEAM TRANSPORT SYSTEM FOR TRANSPORTING ACCELERATED CHARGED PARTICLES OF VARYING ENERGIES FROM AN OUTLET OF AN ACCELERATOR TO A TARGET**
STRAHLENTRANSPORTSYSTEM ZUM TRANSPORT BESCHLEUNIGTER GELADENER TEILCHEN UNTERSCHIEDLICHER ENERGIEN VON EINEM BESCHLEUNIGERAUSLASS ZU EINEM ZIEL
SYSTÈME DE TRANSPORT DE FAISCEAU POUR TRANSPORTER DES PARTICULES CHARGÉES ACCÉLÉRÉES D'ÉNERGIES VARIABLES D'UNE SORTIE D'UN ACCÉLÉRATEUR À UNE CIBLE

(30) Priority: 20.06.2023 EP 23180274
(43) Date of publication of application: 04.03.2026
(73) Proprietor: Ion Beam Applications, 1348 Louvain-la-Neuve (BE)
(72) Inventor: MANDRILLON, Jérôme, 1348 Louvain-la-Neuve (BE)
(74) Representative: Gevers Patents
(86) International application number: PCT/EP2024/065798
(87) International publication number: WO 2024/260754

(56) References cited:
- US-A1- 2012 313 003
- US-A1- 2021 187 328

## Description

### TECHNICAL FIELD

The present invention concerns a charged particle beam therapy system comprising a beam transport system for transporting beams of charged particles from an outlet of an accelerator to a target, wherein the beam transport system is capable of rapidly adapting to transporting the charged particles to the target as their energy is varied. The present invention comprises a bending unit comprising a fixed field dipole for bending the beam, and a steerer located upstream of the bending unit to drive the beams to specific positions and entry angles at an inlet of the bending unit as a function of the energy of the beam, so that the beam is transported to the target irrespective of the energy thereof. The size of the beam transport unit can be reduced, and the quick adaption to beams of varying energies makes it advantageous to implement faster treatments, such as FLASH.

### BACKGROUND OF THE INVENTION

Hadron therapy such as proton therapy is an advantageous radiotherapy treatment of certain tumours, in that doses can be accurately deposited into the tumoural cells to kill them. The TCP / NTCP ratio of the Tumour Control Probability (TCP) to the Normal Tissue Complication Probability (NTCP) is a measure of ratio of the kill rate of tumoural cells to the damage rate to healthy cells. A high value of the TCP / NTCP ratio is desired. Hadron therapy has a higher TCP / NTCP ratio than other radiotherapy techniques, such as X-ray. Charged particles, such as protons or carbon, are accelerated in and extracted from an accelerator. The accelerator can be a cyclotron, a synchrotron, a synchrocyclotron, a LINAC, and the like. The beam of charged particles is transported and guided from the accelerator to the tumoural target in a patient installed in a patient unit through a beam transport system. A gantry is a beam transport unit designed to rotate about the patient to control and vary the angles of irradiation of the target. The angle of rotation can also be controlled and varied with a static beam transport unit by moving the patient / target. Static gantries have also been described. For example, US2021187328 describes a static gantry comprising a toroidal magnet.

Recent work suggests that the TCP / NTCP ratio could possibly be further increased for some tissues by depositing doses at ultra-high deposition rates higher than 1 Gy / s or even higher than 40 Gy / s, in a technique commonly referred to as FLASH. A same dose applied at a ultra-high deposition rate seems to affect healthy cells less than if applied at lower rates, whilst the kill rate of tumoural cells seems to be less sensitive to deposition rate. With the limited performance of existing accelerators, FLASH can advantageously be applied by Pencil Beam Scanning (PBS), by sequentially depositing doses at ultra-high rates according to a specific sequence of a series of subvolumes forming in combination the whole volume of the tumoural target. The doses to be deposited could possibly vary from one subvolume to another and the shift of the beam from one subvolume to a next subvolume of the specific sequence must be quick to maintain the FLASH effect.

The energy E of charged particles is proportional to the square of their velocity v (i.e., E ∝ v²) at the outlet of the accelerator. The velocity v, and thus the energy E of charged particles can be controlled and varied in different ways, depending on the type of accelerator used. Some accelerators can extract particles beams at different energies. For example, synchrotrons and synchrocyclotrons, as described e.g., in EP3876679, and cyclotrons equipped with specific stripper assemblies as described e.g., in EP3503693. In other accelerators, such as cyclotrons which are not equipped with the specific stripper assembly of the type described in EP3503693, the output energy of the particles beams is fixed and cannot be varied. To vary the energy of the particles beams reaching the target, the beam transport system can be equipped with a degrader system comprising one or more blocks of matter that interact with the beam to modulate the energy before reaching the bending unit and the target. The degrader system may require a series of focusing elements to compensate disruption of the transverse beam properties upon traversing the blocks of matter, as well as a concrete shielding structure to retain emitted secondary particles, such as neutrons.

Beam transport systems allow a precise variation of the beam angle reaching the target and a careful control of the beam spot size and position during spot scanning. The bending unit in existing gantries often comprise varying field electromagnets, whose magnetic field amplitude can be varied to steer the particles beams depending on their energies. Varying field electromagnets are, however, complex, quite large and heavy, and are relatively slow to adapt to variations of energies of the charged particles beam, thus increasing treatment times. Longer treatment times are uncomfortable for the patients and, are ill-fitted for treating moving tumours and for implementing FLASH treatments.

US7582886 describes a gantry comprising a bending unit provided with a succession of blocks composed of a of three fixed field dipoles arranged in a succession to alternately focusing and defocusing the particles beam, forming a fixed field alternating gradient (FFAG). Although comprising only fixed field electromagnets, which are simpler and more compact than varying field electromagnets, the gantry described in US7582886 remains very large with a bending radius greater than 3 m (cf. Figure 3 of US7582886) and is composed of a large number of fixed field dipoles positioned side by side, increasing both weight and cost of the gantry.

Tesse et al. presented in Tesse et al., 13th Int. Particle Acc. Conf., IPAC2022, Bangkok, (2022) 2941-2944 an achromatic gantry comprising an FFAG bending unit having smaller dimensions with a theoretical bending radius of 2.1 m. This paper, however, only presents results of an early stage of development, and the described gantry is technically not operational yet., and still maintains relatively large dimensions.

US 2021/0187328 describes a fixed gantry comprising a toroidal magnet formed of a plurality of discrete planar coils spaced apart and extending radially from a primary axis and configured to produce a magnetic field that is periodically symmetric about the primary axis. The fixed gantry equipped with the toroidal magnet is configured to receive beams of charged particles at different radial locations dependent on the momentum to charge ratio of the beam and for focusing the beams to a substantially common point. The fixed gantryod US 2021/0187328 is proposed as a solution to the reduction of size of the rotating gantries, defined in this document as *"extremely large structures of high mechanical rigidity."*

There remains a need for beam transport systems of smaller dimensions and able to rapidly adapt to variations of the energy of the particles beams to ensure that the particles beam reaches the target independently of the energy thereof. A beam transport system more adapted to implement faster treatments to the benefit of the patients comfort, allowing treatment of moving tumours and implementation of FLASH-treatment is also desirable.

### SUMMARY OF THE INVENTION

The present invention is defined in the appended independent claim. Preferred embodiments are defined in the dependent claims. In particular, the present invention concerns a charged particle beam therapy system comprising:
- a patient unit for supporting a patient in a given position.
- an accelerator unit configured for accelerating charged particles and delivering out of an outlet a particles beam of accelerated charged particles of energy between 40 and 450 MeV / u (= MeV per nucleon); the particles are preferably selected among hadrons, more preferably protons, carbon ions, helium ions, and oxygen ions,
- a beam transport system configured for guiding the particles beam of energies varying between 40 and 450 MeV / u, along corresponding beam trajectories centred on a median trajectory starting from the outlet of the accelerator unit to a target located within the patient unit, wherein the beam transport system comprises,
- a bending unit comprising a gap between poles of a fixed field dipole configured for receiving and bending the median trajectory in a bending plane (Y, Z) by an angle comprised between 20 and 270°, preferably between 40 and 140° in the bending plane, between a bending unit inlet and a bending unit outlet), wherein the median trajectory is comprised in the bending plane (Y, Z), with Y ⊥ Z.

The beam transport system further comprises an upstream steerer located upstream of the bending unit and is configured for deflecting the beam trajectories away from the median trajectory by an angle comprised between -15 and +15° in the bending plane (Y, Z). The upstream steerer is configured for providing a deviating function, obtained by creating a uniform magnetic field (B35) in the upstream steerer, wherein a magnitude of the uniform magnetic field (B35) can be controlled to vary an entry position and an entry angle of the beam trajectory at the bending unit inlet (33i) as a function of a beam energy of the particles beam.

The charged particle beam therapy system preferably comprises a processor configured for controlling the amplitude of the uniform magnetic field of the upstream steerer as a function of the beam energy to control the position and the entry angle of the beam trajectory at the bending unit inlet such that to drive the beam trajectory along a curved trajectory of bending radius corresponding to the beam energy and passing through a converging position downstream of the bending unit outlet. The angle of the beam trajectory at the converging position depends on the beam energy. The angle of the beam trajectory relative to the median trajectory at the converging position (330) is preferably but not necessarily independent of the beam energy of the particle beam. In case the beam angle at the converging position varies with the beam energy, the beam transport system can comprise a downstream steerer located at the converging position and configured for deviating the beam trajectories into a common beam trajectory independent of the beam energy.

In a preferred embodiment of the invention, the bending unit can comprise one or more bending subunits disposed along the beam trajectory., Each bending subunit comprises a gap extending along a curved geometry on the bending plane (Y, Z) from a subunit inlet to a subunit outlet and separating first and second magnet coils positioned symmetrically on either side of the bending plane. In one embodiment, the first and second magnet coils can surround first and second magnet poles made of a ferromagnetic material, respectively. In an alternative embodiment, the first and second magnet coils are made of a superconductive material. In both embodiments, the first and second magnet coils are configured for receiving a fixed current, thus forming a fixed field dipole.

The gap preferably has a geometry as follows. In a plane parallel to the bending plane (Y, Z), the gap defines a bend centred on the median trajectory and bounded between a close wall of low radius of curvature and a far wall of large radius of curvature, wherein a distance separating the close wall to the far wall is larger at the bending unit inlet than at the bending unit outlet of the gap. The gap has a width measured parallel to an X-axis normal to the bending plane (i.e., X ⊥ Y ⊥ Z), larger at the close wall than at the far wall. The magnetic field) in the gap (33g) of each bending subunit thus has a highest value in a region adjacent to the far wall, and has a lowest value in a region adjacent close to the close wall. This yields a focussing effect on the particles beam. The highest value of the magnetic field in the gap of each bending subunit is comprised between 1.5 T and 2.6 T, preferably between 1.9 T and 2.4 T, if the first and second magnet coils surround first and second magnet poles made of a ferromagnetic material. In case of superconductive coils, the highest value of the magnetic field can be comprised between 2.5 T and 9 T, preferably between 3.5 T and 6 T.

In a preferred embodiment, the subunit inlet (33i) and / or the subunit outlet (33o) of at least one bending subunit of the bending unit can be not normal to the median trajectory, thus giving more freedom to control a length of the beam trajectory along the gap, and therefore the trajectory angle at the converging position.

The upstream steerer (35u) can be a dipole configured for providing the deviating function only, or for providing deviating function and a 1D-scanning function configured for scanning the beam trajectory in one direction transverse to the beam trajectory to allow scanning the target. Alternatively, the upstream dipole can be an octupole configured to provide the deviating function and one or both of
- a focussing function configured for reducing the size of the particles beam cross-section in one or two directions transverse to the beam trajectory, and / or
- a 1D- or 2D-scanning function configured for scanning the beam trajectory in one or two directions transverse to the beam trajectory to allow scanning the target.

The beam transport system can comprise one or more focusing elements for focusing the accelerated beam along the median trajectory in cross-sectional planes (X, Y) normal to the median trajectory, and preferably a scanner for deflecting the beam trajectories by an angle comprised between -15° and +15° in directions normal to the median trajectory and is configured for controlling the beam trajectories to scan over the target.

The beam transport system of the present invention concerns gantries configured for rotating the bending plane (Y, Z) about a rotating axis passing through the target,. In the latter, the irradiation angles of the target can be varied by moving the patient unit.

### BRIEF DESCRIPTION OF THE FIGURES

For a fuller understanding of the nature of the present invention, reference is made to the following detailed description taken in conjunction with the accompanying drawings in which:
**Figures 1a to 1c****:** show three embodiments of charged particle beam therapy systems according to the present invention.
**Figure 2****:** shows the transverse deviation of the beam trajectories in the bending plane (Y, Z) and perpendicular to the median trajectory for two beam energies relative to the median trajectory as a function of the position along the median trajectory.
**Figure 3a****:** shows a perspective view of a half of part of a bending unit according to the present invention.
**Figures 3b and 3c** show side view on plane (Y, Z) and front view on plane (X, Y) of part of a bending unit according to the present invention.
**Figure 3d** shows beam trajectories of beams of different energies as controlled by the steerer positioned upstream of the bending unit.
**Figures 4a and 4b****:** show two embodiments of quadrupoles, used as focusing units.
**Figures 5a and 5b****:** show two embodiments of octupoles, used as focusing units or as multifunctional upstream or downstream steerers.
**Figures 6a and 6b****:** show a front view and a lateral view of a dipole used as an upstream steerer.

### DETAILED DESCRIPTION OF THE INVENTION

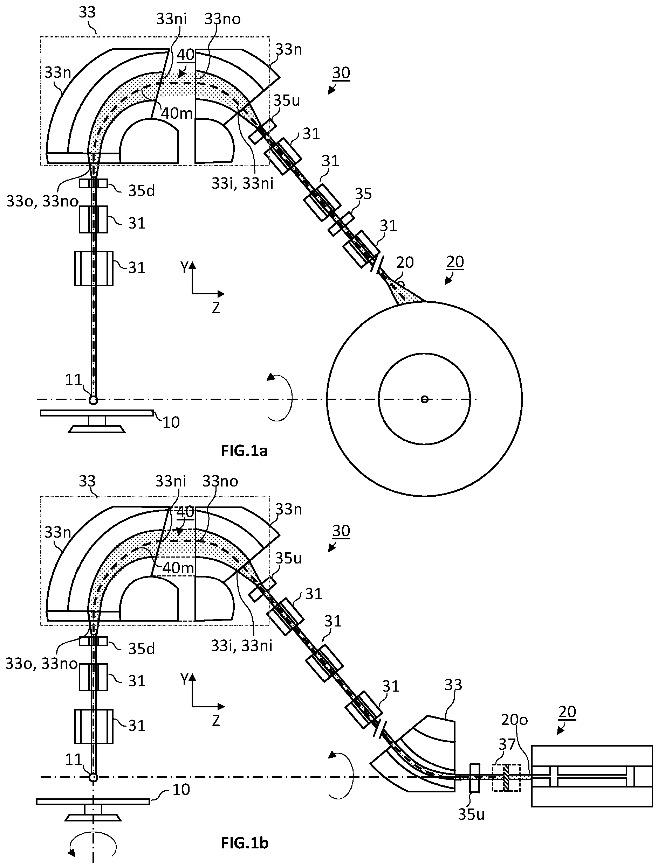

As illustrated in Figures 1a to 1c, the present invention concerns a charged particle beam therapy system comprising a patient unit (10), an accelerator unit (20), and a beam transport system (30) equipped with a bending unit (33).

The patient unit (10) is configured for supporting a patient in a given position. The target (11) is located within the patient unit (10) and includes the tumoural cells of a patient to be irradiated. The position of the patient can be supine or not, and the patient unit (10) can be static or mobile.

The accelerator unit (20) is configured for accelerating charged particles and delivering out of an outlet (20o) a particles beam of accelerated charged particles of energy between 40 and 450 MeV / u (= MeV per nucleon). The charged particles are hadrons, preferably protons, carbon ions, helium ions, or oxygen ions. The accelerator unit (20) can be of the type allowing varying the energy of the output particles beam, such as a synchrotron, a LINAC, or of the type exiting particles beam of fixed energy. In the latter case, the beam transport system (30) must comprise a degrader unit discussed in continuation.

The beam transport system (30) is configured for guiding the particles beam of energies (Ej, j = 1 to 3) varying between 40 and 450 MeV / u, along corresponding beam trajectories (40e) centred on a median trajectory (40m) starting from the outlet (20o) of the accelerator unit (20) to a target (11) located within the patient unit (10). The bending unit (33) comprises a gap (33g) between poles (33p) of a fixed field dipole extending from a beam bending unit inlet (33i) to a bending unit outlet (33o). The magnetic field (B33) in the gap (33g) is therefore not varied to modify the bending radius of beam trajectories (40e) as a function of the beam energy (Ej). The bending unit is configured for receiving and bending the median trajectory (40m) in a bending plane (Y, Z) by an angle comprised between 20 and 270°, preferably between 40 and 140° in the bending plane, between the bending unit inlet (33i) and the bending unit outlet (33o), wherein the median trajectory (40m) is comprised in the bending plane (Y, Z), with Y ⊥ Z. The beam trajectories of particles beams of all energies compatible with the charge particle beam therapy system are comprised in the bending plane (Y, Z) and are enclosed within the gap (33g) geometry.

The gist of the present invention is to include in a first bending module an upstream steerer (35u) located upstream of the bending unit (33), wherein upstream and downstream are defined relative to the beam travelling direction. The upstream steerer (35u) is configured for deflecting the beam trajectories (40e) away from the median trajectory (40m) by an angle comprised between -15 and +15° in the bending plane (Y, Z). A magnitude of the uniform magnetic field (B35) of the upstream steerer (35u) can be varied to vary a position and an entry angle of the beam trajectory at the bending unit inlet (33i) as a function of the beam energy (Ej, j = 1 to 3).

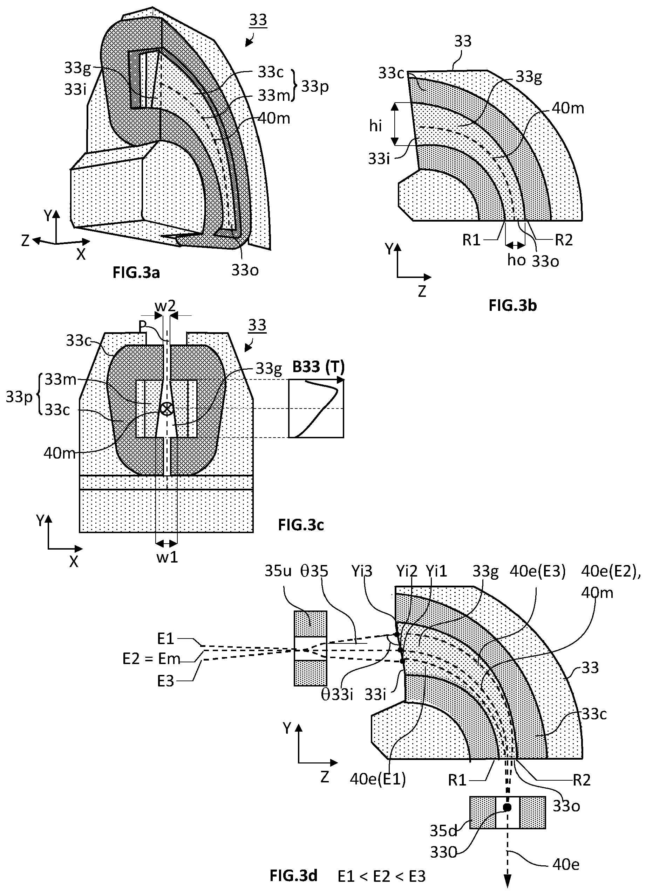

The upstream steerer (35u) is configured for providing a deviating function. The deviating function is obtained by creating a uniform magnetic field (B35) within a volume of interest. The amplitude of the uniform magnetic field (B35) of the upstream steerer (35u) as a function of the beam energy (Ej) is preferably controlled by a processor (not shown). Controlling the entry position (Yij) and the entry angle (θ33i) of the beam trajectory at the bending unit inlet (33i) allows driving the beam trajectory (40e) along a curved trajectory of bending radius dependent on the beam energy (Ej) and passing through a converging position (330) downstream of the bending unit outlet (33o). An angle of the beam trajectory (40e) relative to the median trajectory (40m) at the converging position (330) is preferably independent of the beam energy (Ej) of the particle beam. As shown in Figure 3d, however, in case the angle of the beam trajectory (40e) at the converging position (330) depends on the beam energy (Ej), then the beam transport system (40) can comprise a downstream steerer (35d) located at the converging position (330) and configured for deviating the beam trajectories into a common beam trajectory independent of the beam energy (Ej).

As shown in Figures 1a to 1c, upstream and downstream of the bending unit (33) the beam transport system (40) can comprise one or more focussing elements (31) and even steerers (35) to correct any degradation of the beam properties, as long as the upstream steerer (35u) is provided upstream of the bending unit inlet (33i). The beam transport system (40) can also comprise a scanner configured for controlling the beam trajectories to scan over the target (11), in particular in a PBS treatment. The upstream steerer (35u) must fulfil the primary function of deviating the beam trajectories depending on the beam energy (Ej) to reach the bending unit inlet (33i) at the entry position (Yij) and entry angle (θ33i) corresponding to the beam energy (Ej). In certain embodiments, the upstream steerer (35u) can fulfil additional functions, such as focussing and even scanning the particles beam. This is advantageous, as the upstream steerer (35u) can thus replace focussing elements and scanners which would otherwise be required and so reduce the overall size, weight, and cost of the beam transport unit (30).

### BENDING UNIT (33)

The bending unit (33) according to the present invention does not comprise any varying field electromagnet. It comprises only fixed field dipoles that have dipolar field components. Fixed field dipoles can be permanent magnets or electromagnets whose magnetic field cannot be varied (fixed current is supplied to the coils), as illustrated in Figures 3a to 3d. The fixed field electromagnets are substantially smaller, simpler and cheaper than varying field electromagnets. They also allow reaching higher values of the magnetic field (B33) in the gap (33g). Not varying the magnetic field in the bending unit (33) also has the advantage of yielding a quicker adaption of the optics to variations of the beam energy (Ej) than with varying field electromagnets.

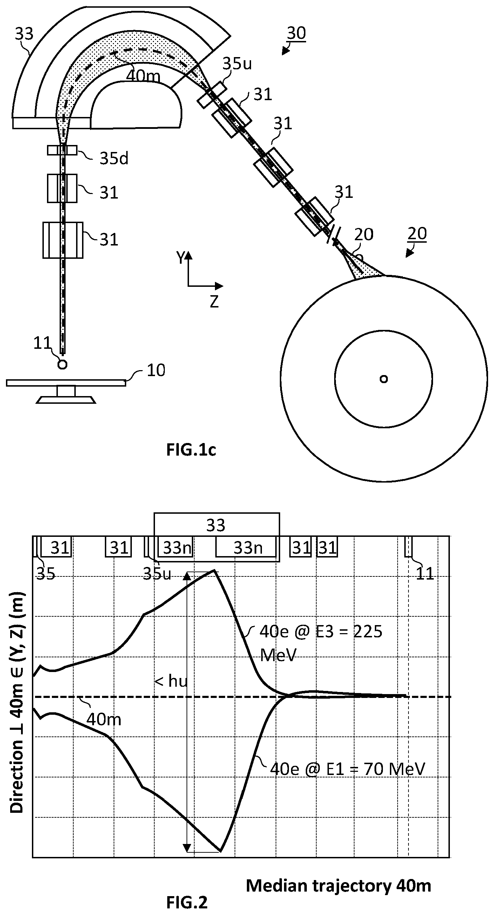

In a preferred embodiment of the present invention, the bending unit (33) comprises one or more bending subunits (33n) disposed along the beam trajectory. Figures 1a and 1b show bending units of this type, formed by two bending subunits (33n), whilst Figure 1c shows a bending unit formed by a single element (or a single bending subunit (33n)). Splitting the bending unit (33) into two (or more) bending subunits (33n) can have the advantage of allowing a safety feature to be inserted between the two (or more) bending subunits (33n) configured for interrupting the particles beam in case the beam trajectory was not comprised within safety boundaries. It can also ease access to the elements of the bending unit in case of repair or maintenance. Finally, it is possible, albeit not necessary, to insert focussing elements (31) between the two (or more) bending subunits (33n) in case excessive excursions of the particles in the transverse directions X and Y occurred. A scanner can also be positioned between two bending subunits (33n). On the other hand, a bending unit (33) formed by a single element as shown in Figure 1c is more compact, reducing the overall size of the charged particle beam therapy system.

As shown in Figures 3a to 3d, each bending (sub)unit (33, 33n) comprises a gap (33g) extending along a curved geometry on the bending plane (Y, Z) from a (sub)unit inlet (33i, 33ni) to a (sub)unit outlet (33o, 33no). The subunit inlet (33ni) of the bending subunit (33n) located most upstream of the bending unit (33) forms the bending unit inlet (33i), and the subunit outlet (33no) of the bending subunit (33n) located most downstream of the bending unit (33) forms the bending unit outlet (33o). The positions of the upstream steerer (35u) and of the optional downstream steerer (35d) are therefore defined relative to the bending unit inlet (33i) and outlet (33o) as defined supra for bending units composed by one or by more than one bending subunits (33n). The number of bending subunits (33n) forming the bending unit (33) is preferably one or two.

The gap (33g) separates first and second magnet coils (33c) positioned symmetrically on either side of the bending plane. In a preferred embodiment, the first and second magnet coils (33c) surround first and second magnet poles (33p) made of a ferromagnetic material, respectively. A maximum value of magnetic field (B33) in the bending plane and in the gap (33g) of the downstream fixed field dipole (33n) can be larger than 1.8 T and is preferably lower than 2.4 T

In an alternative embodiment, the first and second magnet coils (33c) are made of a superconductive material. In both embodiments, the current driven to the magnet coils is constant (and independent of the beam energy (Ej)).

The bending unit's prime function is to bend the particles trajectories (40e). To achieve this prime function, in a plane parallel to the bending plane (Y, Z) as shown in Figure 3b, the gap (33g) defines a bend centred on the median trajectory (40m) and bounded between a close wall of lower radius of curvature (R1) and a far wall of larger radius of curvature (R2) (R1 < R2). The distance (hi, ho) separating the close wall from the far wall can be larger at the bending unit inlet (33i) than at the bending unit outlet (33o) of the gap (33g), to yield a larger range of entry positions (Yij) for the particles beam.

In a preferred embodiment, the bending unit also has a second function of focussing the particles beam as it travels along the curved gap (33g) by the geometry of the latter. In this embodiment as shown in Figure 3c, the gap (33g) has a width (w1, w2) larger at the close wall than at the far wall (w1 > w2), measured along transverse planes (X, Y) normal to the median trajectory (40m). As shown in the inset of Figure 3c, the magnetic field (B33) in the gap (33g) of each bending subunit (33n) has a highest value in a region adjacent to the far wall, and has a lowest value in a region adjacent close to the close wall. This magnetic field gradient has the effect of focussing the particles beam in a direction transverse to the median trajectory (40m) and in the bending plane as it travels along the gap (33g). This geometry of the gap (33g) can have a defocussing effect on the particles beam in a direction normal to the bending plane (Y, Z), but it remains a preferred embodiment because it reduces the deviations between extreme energies in the bending plane (Y, Z).

The highest value of the magnetic field (B33) in the gap (33g) of each bending subunit (33n) can be comprised,
- between 1.5 T and 2.6 T, preferably between 1.9 T and 2.4 T, if the first and second magnet coils (33c) surround first and second magnet poles (33p) made of a ferromagnetic material, or
- between 2.5 T and 9 T, preferably between 3.5 T and 6 T if the first and second magnet coils (33c) are superconductive.

A radius of the median trajectory (40m) in the gap (33g) can be as small as about 1000 mm, rendering the beam transport system (30) quite compact/

### UPSTREAM STEERER (35u)

As illustrated in Figure 3d, the primary function of the upstream steerer is to drive the particles beam towards a specific entry position (Yij, j = 1 to 3) and entry angle (*θ*33i) at the bending unit inlet (33i) corresponding to the beam energy (Ej) ensuring that, under the effect of the fixed field dipole(s) of the bending unit (33), the particles beam follows a bending trajectory leading to a converging position (330) located downstream of the bending unit outlet (33o), regardless of the beam energy (Ej). A deviating function suffices for the upstream steerer (35u) to fulfil the primary function of the steerer. The deviating function is obtained by generating a uniform magnetic field (B35) between the two poles of a dipole. The entry position (Yij) and entry angle (*θ*33i) at the bending unit inlet (33i) of the beam trajectory is controlled as a function of the energy (Ej) of the particles beam by varying the uniform magnetic field (B35) in the upstream steerer (35u), created by varying field electromagnets.

It is preferred that the particles beam exits through the bending unit outlet (33o) with a fixed exit angle independent of the energy (Ej) of the particles beam and crosses the converging position (330) along a fixed trajectory. If, however, as illustrated in Figure 3d, this is not the case, then a downstream steerer (35d) can be provided, located at the converging position (330) to realign the converging particles beams to form a fixed trajectory downstream of the downstream steerer (35d). It is important to be able to control accurately the trajectory of the particles beam downstream of the bending unit (33), since the target (11) is close, and the particles beam must accurately reach selected points of the target (11).

The upstream steerer (35u) is configured for deflecting the beam trajectories (40e) away from the median trajectory (40m) by an angle (θ35) comprised between -15 and +15° in the bending plane (Y, Z). This is achieved by varying a magnitude of the uniform magnetic field (B35) of the upstream steerer (35d) to control the entry position (Yij) and the entry angle (θ33i) of the beam trajectory at the bending unit inlet (33i) as a function of the beam energy (Ej) of the particles beam (40e). If the bending unit inlet (33i) is parallel to the outlet of the upstream steerer (35u), then θ33i = θ35. In the embodiment illustrated in Figure 3d, this is, however, not the case and θ33i ≠ θ35.

As illustrated in Figure 3d, the deviating function of the upstream steerer (35u) is required for bending the beam trajectories to drive them to the bending unit inlet (33i) at the entry position (Yij) with the entry angle (θ33i) corresponding to the beam energy (Ej). The deviating function is achieved by forming and controlling the magnitude of the uniform magnetic field (B35).

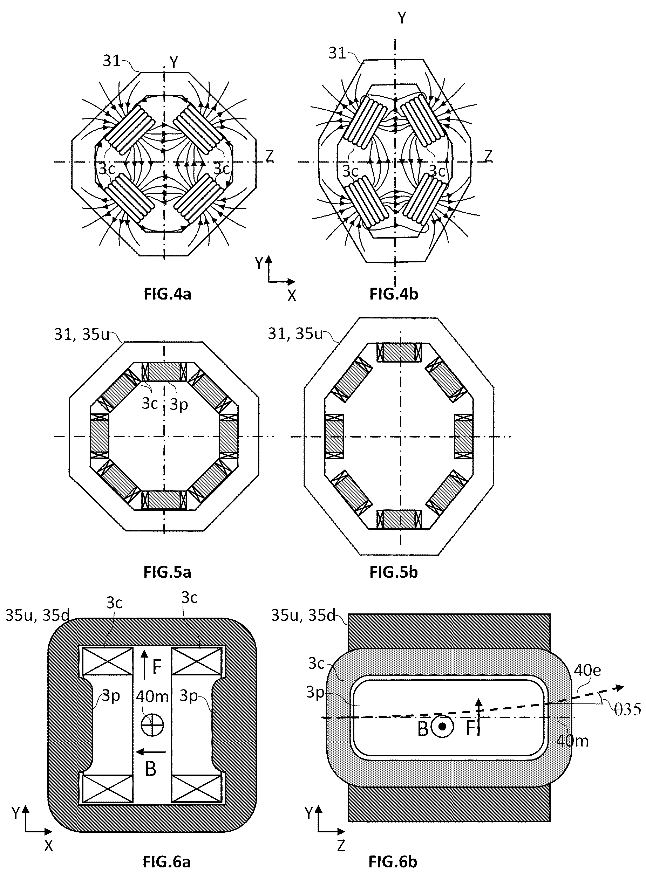

The uniform magnetic field can be created by any multipole having a degree of symmetry of two, such as a dipole illustrated in Figures 6a and 6b, a quadrupole illustrated in Figures 4a and 4b, a hexapole (not illustrated) and an octupole as illustrated in Figures 5a and 5b, provided the currents are correctly driven in the corresponding coils (3c). If the upstream steerer (35u) is used to yield the deviating function only, then it is preferably a dipole as illustrated in Figures 6a and 6b. Note that a dipole can ensure the deviating function as well as a scanning function in one direction only (here in the bending plane (Y, Z) and perpendicular to the median trajectory (40m) = 1D-scanning function). In this case, using a quadru- or octupole would not be necessary, as they are more complex, bulkier, and more expensive than dipoles.

An octupole can be used as upstream steerer (35u) if, beside the deviating function, the upstream steerer (35u) should yield a further function, such as a focussing function and / or scanning function in one or two directions on a plane normal to the median trajectory (40m) (= 1D- or 2D-scanning function). Referring to Figures 5a and 5b showing two embodiments of octupoles, the deviating function of deviating the particles beam along the Y-axis can be fulfilled by driving current into at least the pair of facing magnet elements forming a dipole aligned on the X-axis. The scanning function can be obtained by controlling the current in the dipole aligned along the Y-axis. The focussing function can be obtained by conveniently driving current in the four remaining magnet elements forming a quadrupole. It is therefore possible to combine several functions, necessarily including the deviating function, in the single upstream steerer (35u) which can be advantageous in terms of size and weight of the beam transport system (30). It should be noted that, as well known to the skilled person, the functions of quadru- and octupoles depend on the direction of circulation of the current in the corresponding coils (3c) of the different magnet elements.

To summarise, in a preferred embodiment, the upstream steerer (35u) can either be,
- a dipole configured for providing the deviating function only,
- a dipole configured for providing the deviating function and a 1D-scanning function configured for scanning the beam trajectory in one direction transverse to the beam trajectory to allow scanning the target (11), or
- an octupole configured for providing, besides the deviating function, also,
   ∘ a focussing function configured for reducing the size of the particles beam cross-section in one or two directions transverse to the beam trajectory, and / or
   ∘ 1D- or 2D- a scanning function configured for scanning the beam trajectory in one or two directions transverse to the beam trajectory to allow scanning the target (11).

The uniform magnetic field (B35) required for providing the deviating function can be varied between ± 2 T, preferably ± 1 T, depending on the length of the upstream steerer (35u) measured along the median trajectory (40m), for deviating by an angle of ± 15° high energy particles beams, such as the particles beam 40e(E3) of energy E3 illustrated in Figure 3d, and low energy particles beams, such as the particles beam 40e(E1) of energy E1. A uniform magnetic field of 0 T would not deviate the beam trajectory and it can preferably be applied in case the particles beam has a median energy (Em) following the median trajectory, as illustrated in Figure 3d with the particles beam of energy, E2 = Em. Of course, no 1D- or 2D-scanning function is possible with a uniform magnetic field of 0 T.

### FIRST BENDING MODULE FORMED BY UPSTREAM STEERER (35u) AND BENDING UNIT (33)

The gist of the present invention is the first bending module, combining a bending unit (33) comprising one or more fixed field dipoles (33n), with an upstream steerer (35u) located upstream of the bending unit (33) as discussed supra. Depending on the treatment plan, a particles beam is transported along the beam transport system (30) reaching the upstream steerer (35u) with a given energy (Ej). It is preferred that the particles beam reaches the upstream steerer (35u) at a same entry point and with a same orientation regardless of the beam energy (Ej), but this is not essential, and the particles beam could reach the upstream steerer (35u) at different entry points and / or with different orientations depending on the beam energy (Ej). Figure 3d shows a specific embodiment of three particles beams of energies E1, E2, E3, with E1 < E2 < E3, reaching the upstream steerer (35u) at a same entry point but with different orientations depending on the beam energy (Ej). Regardless of the particles beam entry point and orientation at the upstream steerer (35u) it is essential that the uniform magnetic field (B35) in the upstream steerer (35u) be controlled such as to deviate the beam trajectories according to their energies (Ej) such that the particles beams reach the bending unit inlet (33i) at the specific entry position (Yij) with specific entry angle (θ33i) allowing the particles beam to follow a corresponding bending trajectory in the bending unit (33) allowing the particles beam to converge at the converging position (330) located downstream of the bending unit outlet (33o). Control of the uniform magnetic field (B35) in the upstream steerer is preferably carried out by a processor.

The radius of curvature (r) of the bending trajectory in each bending subunit (33n) increases with the energy (Ej) (velocity (v)) and is inversely proportional to the magnetic field (B) in the gap (33g),, so that the radius of curvature of a particles beam of low energy (E1) is smaller than the one of a particles beam of high energy (E3), with E1 < E3. The gap (33g) of each bending subunit (33n) must therefore carefully be designed to accommodate beam trajectories of particles beams of energy (Ej) ranging from maximum to minimum values, defining the limits of energy tolerance of the beam transport unit (30). It also follows, as illustrated in Figure 3d, that the upstream steerer is configured to deviate the beam trajectory of a particles beam of lower energy (E1) such that the beam trajectory reaches the bending unit inlet (33i) at the entry position (Yi1) located closer to the close wall of low radius of curvature (R1). Inversely, the upstream steerer is configured to deviate the beam trajectory of a particles beam of higher energy (E3) such that the beam trajectory reaches the bending unit inlet (33i) at the entry position (Yi3) located closer to the far wall of large radius of curvature (R3). A length (hi) of the gap (33g) at the bending unit inlet (33i) measured along the Y-axis must be sufficient to accommodate the entry positions (Yij) of the beam trajectories of all energies ranging within the limits of energy tolerance of the beam transport unit (30).

The exit position and exit angle relative to the median trajectory (40m) of the beam trajectories (40e) depends of course on the radius of curvature of the beam trajectory in a bending subunit (33n) but also on the length of the beam trajectory in the gap (33g). The length of the beam trajectory in the gap (33g) depends *inter alia* on the entry angle (θ33i), which itself depends inter alia on the orientation of the bending unit inlet (33i). To give further design freedom to control the distance of the section of beam trajectories (40e) included in the gap (33g) (i.e., between the bending unit inlet (33i) and the bending unit outlet (33o)) as a function of the beam energy (Ej), the bending unit inlet (33i) can be non-perpendicular to the median trajectory (40m). In the bending plane (Y, Z), the bending unit inlet (33i) can be non-straight, e.g., curved or segmented. In the embodiment illustrated in Figure 3d, this distance is lengthened for particles beams of higher energies (E3) and shortened for particles beams of lower energies (E1), and thus affecting the entry angle (θ33i) and beam trajectory length in the gap, accordingly. Similarly, the beam trajectory length in the gap (33g) can also be modified by controlling the geometry of the bending (sub)unit outlet (33o, 33no) such that the particles beam exits the bending (sub)unit with a non-perpendicular angle with the bending (sub)unit outlet (33o, 33no).

When the energy (Ej) of the particles beam to be transported in the beam transport system (30) is changed, the magnetic field (B33) of the bending unit (33) can be maintained fixed and only the current fed to the upstream steerer (35u) is varied to control the uniform magnetic field (B35) the particles beam must traverse prior to reaching the bending unit inlet (33i) at the desired position (Yij) and with the correct orientation (entry angle (θ33i) to follow the desired bending trajectory through the bending unit and exiting the bending unit (33) to converge towards the converging position (330). This allows a substantially quicker adaption of the beam transport system (30) to the beam energy (Ej), than with conventional varying field bending units.

The combination of the upstream steerer (35u) with a fixed field bending unit of the type FFAG is possible, but it is preferred to use a bending unit (33) as defined supra. Indeed, the present invention does not require an alternating gradient since the beam trajectory as a function of the beam energy (Ej) is controlled by the upstream steerer. One advantage of FFAG is that the particles beam is being constantly focussed as it crosses successive three block-modules of [focussing - defocussing - focussing] dipoles. A focussing function can also be achieved, on the one hand, by controlling the profile of the magnetic field (B33) in the gap (33g), as discussed supra, e.g., with the design of the gap (33g), and / or with a quadrupole positioned upstream of the bending unit (33), and / or by the upstream steerer (35u) if an octupole is used. The bending unit (33) as discussed supra is more compact than an FFAG and is therefore preferred.

If the bending unit (33) is formed of more than one subunit (preferably not more than two subunits (33n)), it is preferred that the subunits (33n) be directly adjacent to one other. In some cases, a focussing element (31) or a scanner can be interposed between two bending subunits.

Similarly, it is preferred that the upstream steerer (35u) be directly adjacent to the bending unit (33) as shown in Figure 3d. It is, however, possible to interpose a focussing element (31) or a scanner between the upstream steerer (35u) and the bending unit (33).

### FOCUSSING ELEMENTS (31)

Focussing elements (31) are required to ensure the integrity of the particles beam properties when it reaches the target (11). This is particularly true when a degrader system (37) is used, as the interaction of the particles beam with the blocks of matter disrupts the integrity of the particles beam properties. Focussing elements (31) are well known to the skilled person, and their uses and functions in the present invention do not differ from the prior art.

As illustrated in Figures 4a & 4b and 5a & 5b, focussing elements can be quadrupoles or octupoles. The coils in the quadrupole or octupole can be equidistant as shown in Figures 4a and 5a, or they can be equidistant two by two, as shown in Figures 4b and 5b. The latter geometry yields a higher tolerance to the particles beam size (in the Figures, along the Y-axis). By correctly driving current in the various coils, the magnetic field thus created focusses the beam in one or both directions normal to the beam trajectory.

### BEAM TRANSPORT SYSTEM (30)

The most commonly used beam transport systems are gantries, configured for rotating about an axis passing by the target (11) as shown in Figures 1a and 1b. In some cases, however, the beam transport system can be static. To vary the orientation of the particles beam relative to the target, the target can be moved instead. The present invention applies to both gantries and static beam transport systems.

The gantries can rotate together with the accelerator (20), as shown in Figure 1a. The particles beam therefore does not rotate relative to the accelerator (20), which has the advantage of substantially simplifying the design of the gantry which needs no "hinge" to rotate the gantry relative to the accelerator (20).

The gantry can also rotate relative to the accelerator (20), as illustrated in Figure 1b. In this case a "hinge" is required to allow this rotation of the gantry with particles beams of different beam energies (Ej). The "hinge" can be provided by a second bending module formed by an upstream steerer (35u) or a downstream steerer associated with a bending unit (33). Figure 1b illustrates an embodiment of a second bending module comprising an upstream steerer (35u). The principle of the second bending module is exactly the same as with the first bending module discussed supra, but the second bending module is located closer to the accelerator (20) when the first bending module discussed supra is located closer to the target (11).

As shown in Figure 1b, it the accelerator is configured for emitting a particles beam of fixed exit energy, the beam transport system (30) can be equipped with a degrader system (37), to absorb predefined fractions of the exit energy, to control the beam energy (Ej) reaching the target. Degrader systems are well known in the art and may require a series of focusing elements to compensate disruption of the transverse beam properties upon traversing the blocks of matter, as well as a concrete shielding structure to retain emitted secondary particles, such as neutrons. For sake of clarity, all these elements are represented in the single box labelled (37) of Figure 1b. For accelerators configured for extracting particles beams of varying energies, a degrader system (37) is not required, allowing the overall size of the beam transport system to be reduced accordingly.

Both upstream and downstream of the first bending module, focussing elements (31) can be arranged to control the transverse properties of the beam along the beam transport system (30). These are well known in the art and need not be further described herein.

Figure 2 shows transverse deviations of the beam trajectories (40e) (40m) in the bending plane (Y, Z) and perpendicular to the median trajectory (40m) for beam energies, E1 = 70 MeV, and E3 = 225 MeV relative to the median trajectory. as a function of the position along the median trajectory (40m). Transverse deviation of the beam trajectories (40e) are allowed between the accelerator outlet (20o) and the target (11) (both excluded) as long as they all converge to converging position (330) and further to the desired spots in the target (11).. It can be seen in Figure 2, that thebeam trajectories (40e) of different energies (Ej) follow substantially different beam trajectories as long as they are being transported in the beam transport system (30), but they must merge as they reach the target (11).

| **#** | **Definition** |
|---|---|
| | |
| 3c | Coil |
| 3p | Magnet pole |
| 10 | Patient unit |
| 11 | Target |
| 20 | Charged particles accelerator |
| Z0o | Accelerator outlet |
| 30 | Beam transport system |
| 31 | Focusing element |
| 33 | Bending unit |
| 33c | Coil of bending magnet |
| 33i | Bending unit inlet |
| 33g | gap |
| 33n | Bending subunit |
| 33ni | Bending subunit inlet |
| 33no | Bending subunit outlet |
| 33o | Bending unit outlet |
| 33p | Magnet pole of bending unit |
| 35 | Steerer |
| 35d | Downstream steerer |
| 35u | Upstream steerer |
| 37 | Degrader system |
| | |
| B33 | Magnetic field in gap of bending unit |
| B35 | Magnetic field in upstream steerer |
| Yij | Entry position of particles beam at the bending unit inlet |
| (X, Y) | Bending plane |
| θ33i | Entry angle to bending unit inlet of particles beam relative to median trajectory |
| θ35 | Exit angle from upstream steerer of particle beam relative to median trajectory |
| | |

## Claims

1. A charged particle beam therapy system comprising:
• a patient unit (10) for supporting a patient in a given position.
• an accelerator unit (20) configured for accelerating charged particles and delivering out of an outlet (20o) a particles beam of accelerated charged particles of energy between 40 and 450 MeV / u (= MeV per nucleon),
• a beam transport system (30) configured for guiding the particles beam of energies (Ej, j = 1 to 3) varying between 40 and 450 MeV / u, along corresponding beam trajectories (40e) centred on a median trajectory (40m) starting from the outlet (20o) of the accelerator unit (20) to a target (11) located within the patient unit (10), wherein the beam transport system (30) comprises,
• a bending unit (33) comprising a gap (33g) between poles (33p) of a fixed field dipole configured for receiving and bending the median trajectory (40m) in a bending plane (Y, Z) by an angle comprised between 20 and 270°, preferably between 40 and 140° in the bending plane, between a bending unit inlet (33i) and a bending unit outlet (33o), wherein the median trajectory (40m) is comprised in the bending plane (Y, Z), with Y ⊥ Z,
**characterized in that,**
• the beam transport system (30) is a gantry configured for rotating the bending plane (Y, Z) about a rotating axis passing through the target (11),
• the beam transport system (30) further comprises an upstream steerer (35u) located upstream of the bending unit (33) and is configured for deflecting the beam trajectories (40e) away from the median trajectory (40m) by an angle (θ35) comprised between -15 and +15° in the bending plane (Y, Z), and **in that,**
• the upstream steerer (35u) is configured for providing a deviating function, obtained by creating a uniform magnetic field (B35) in the upstream steerer (35u), wherein a magnitude of the uniform magnetic field (B35) can be controlled to vary an entry position (Yij, with j = 1 to 3)) and an entry angle (θ33i) of the beam trajectory at the bending unit inlet (33i) as a function of a beam energy (Ej, with j = 1 to 3) of the particles beam.

2. Charged particle beam therapy system according to claim 1, comprising a processor configured for controlling the amplitude of the uniform magnetic field (B35) of the upstream steerer (35u) as a function of the beam energy (Ej), to control the position (Yij) and the entry angle (θ33i) of the beam trajectory at the bending unit inlet (33i) such that to drive the beam trajectory (40e) along a curved trajectory of bending radius corresponding to the beam energy (Ej), and passing through a converging position (330) downstream of the bending unit outlet (33o), wherein an angle of the beam trajectory (40e) relative to the median trajectory (40m) at the converging position (330) is preferably independent of the beam energy (Ej) of the particle beam.

3. Charged particle beam therapy system according to claim 2, wherein the angle of the beam trajectory (40e) at the converging position (330) depends on the beam energy (Ej) and wherein the beam transport system (30) comprises a downstream steerer (35d) located at the converging position (330) and configured for deviating the beam trajectories into a common beam trajectory independent of the beam energy (Ej).

4. Charged particle beam therapy system according to any one of claims 1 to 3, wherein the bending unit (33) comprises one or more bending subunits (33n) disposed along the beam trajectory, wherein each bending subunit (33n) comprises a gap (33g) extending along a curved geometry on the bending plane (Y, Z) from a subunit inlet (33ni) to a subunit outlet (33no) and separating first and second magnet coils (33c) positioned symmetrically on either side of the bending plane, wherein either,
• the first and second magnet coils (33c) surround first and second magnet poles (33p) made of a ferromagnetic material, respectively, or
• the first and second magnet coils (33c) are made of a superconductive material.

5. Charged particle beam therapy system according to claim 4, wherein the gap (33g) is defined as follows,
• in a plane parallel to the bending plane (Y, Z), the gap (33g) defines a bend centred on the median trajectory (40m) and bounded between a close wall (33gc) of low radius of curvature (R1) and a far wall of large radius of curvature (R2) (R1 < R2), wherein a distance (hi, ho) separating the close wall to the far wall is larger at the bending unit inlet (33i) than at the bending unit outlet (33o) of the gap (33g),
• the gap (33g) has a width (w1, w2) measured parallel to an X-axis normal to the bending plane (i.e., X ⊥ Y ⊥ Z), larger at the close wall than at the far wall (w1 > w2).

6. Charged particle beam therapy system according to any one of the preceding claims, wherein a magnetic field (B33) in the gap (33g) of each bending subunit (33n) has a highest value in a region adjacent to the far wall, and has a lowest value in a region adjacent close to the close wall.

7. Charged particle beam therapy system according to claim 6, wherein the highest value of the magnetic field (B33) in the gap (33g) of each bending subunit (33n) is comprised,
• between 1.5 T and 2.6 T, preferably between 1.9 T and 2.4 T, if the first and second magnet coils (33c) surround first and second magnet poles (33p) made of a ferromagnetic material, or
• between 2.5 T and 9 T, preferably between 3.5 T and 6 T if the first and second magnet coils (33c) are superconductive.

8. Charged particle beam therapy system according to any one of claims 4 to 7, wherein a subunit inlet (33i) and / or a subunit outlet (33o) of at least one bending subunit of the bending unit (33) is not normal to the median trajectory (40m).

9. Charged particle beam therapy system according to any one of the preceding claims, wherein the particles are selected among hadrons, preferably protons, carbon ions, helium ions, and oxygen ions.

10. Charged particle beam therapy system according to any one of claims 1 to 9, wherein the upstream steerer (35u) is either,
• a dipole configured for providing the deviating function only, or
• a dipole configured for providing deviating function and a 1D-scanning function configured for scanning the beam trajectory in one direction transverse to the beam trajectory to allow scanning the target.
• an octupole configured for providing the deviating function and,
∘ a focussing function configured for reducing the size of the particles beam cross-section in one or two directions transverse to the beam trajectory, and / or
∘ a 1D- or 2D-scanning function configured for scanning the beam trajectory in one or two directions transverse to the beam trajectory to allow scanning the target (11).

11. Charged particle beam therapy system according to any one of the preceding claims, wherein the beam transport system (30) comprises,
• one or more focusing elements (31) for focusing the accelerated beam along the median trajectory (40m) in cross-sectional planes (X, Y) normal to the median trajectory (40m), and
• preferably a scanner for deflecting the beam trajectories by an angle comprised between -15° and +15° in directions normal to the median trajectory (40m) and is configured for controlling the beam trajectories to scan over the target (11).

## Patentansprüche

1. Geladene-Teilchen-Strahl-Therapiesystem, welches Folgendes umfasst:
• eine Patienteneinheit (10) zur Halten eines Patienten in einer gegebenen Position,
• eine Beschleunigereinheit (20), die zum Beschleunigen geladener Teilchen und zum Abgeben eines Teilchenstrahls beschleunigter geladener Teilchen mit einer Energie zwischen 40 und 450 MeV / u (= MeV pro Nukleon) aus einem Auslass (20o) konfiguriert ist,
• ein Strahltransportsystem (30), das zum Leiten des Teilchenstrahls mit Energien (Ej, j = 1 bis 3), die zwischen 40 und 450 MeV / u variieren, entlang entsprechender Strahltrajektorien (40e), die um eine Mediantrajektorie (40m) zentriert sind, beginnend von dem Auslass (20o) der Beschleunigereinheit (20) zu einem Ziel (11), das sich innerhalb der Patienteneinheit (10) befindet, konfiguriert ist, wobei das Strahltransportsystem (30) Folgendes umfasst:
• eine Biegeeinheit (33), die einen Spalt (33g) zwischen Polen (33p) eines Festfelddipols umfasst und die zum Empfangen und Biegen der Mediantrajektorie (40m) in einer Biegeebene (Y, Z) um einen Winkel zwischen 20 und 270°, vorzugsweise zwischen 40 und 140° in der Biegeebene, zwischen einem Biegeeinheitseinlass (33i) und einem Biegeeinheitsauslass (33o) konfiguriert ist, wobei die Mediantrajektorie in der Biegeebene (40m) in der Beigeebene (Y, Z), mit Y ⊥ Z, enthalten ist, **dadurch gekennzeichnet, dass**
• das Strahltransportsystem (30) eine Gantry ist, die zum Drehen der Biegeebene (Y, Z) um eine Rotationsachse konfiguriert ist, die durch das Ziel (11) verläuft,
• das Strahltransportsystem (30) ferner ein stromaufwärts gelegenes Lenkelement (35u) umfasst, das sich stromaufwärts von der Biegeeinheit (33) befindet und dazu konfiguriert ist, die Strahltrajektorien (40e) relativ zu der Mediantrajektorie (40m) um einen Winkel (θ35), der zwischen -15 und +15° beträgt, in der Biegeebene (Y, Z) abzulenken, und dadurch, dass,
• das stromaufwärts gelegene Lenkelement (35u) zum Bereitstellen einer Ablenkfunktion konfiguriert, die durch Erzeugen eines einheitlichen Magnetfelds (B35) in dem stromaufwärtsgelegenen Lenkelement (35u) erhalten wird, wobei ein Betrag des einheitlichen Magnetfeldes (B35) gesteuert werden kann, um eine Eintrittsposition (Yij, mit j = 1 bis 3) und einen Eintrittswinkel (θ33i) der Strahltrajektorie an dem Biegeeinheitseinlass (33i) als Funktion einer Strahlenergie (Ej, mit J = 1 bis 3) des Teilchenstrahls zu variieren.

2. Geladene-Teilchen-Strahl-Therapiesystem nach Anspruch 1, das einen Prozessor umfasst, der zum Steuern der Amplitude des einheitlichen Magnetfelds (B35) des stromaufwärts gelegenen Lenkelements (35u) als eine Funktion der Strahlenergie (EJ) konfiguriert ist, um die Position (Yij) und den Eintrittswinkel (θ33i) der Strahltrajektorie an dem Biegeeinheitseinlass (33i) so zu steuern, dass die Strahltrajektorie (40e) entlang einer gekrümmten Trajektorie mit einem Biegeradius angetrieben wird, der der Strahlenergie (Ej) entspricht, und durch eine Konvergenzposition (330) stromabwärts von dem Biegeeinheitsauslass (33o) verläuft, wobei ein Winkel der Strahltrajektorie (40e) relativ zu der Mediantrajektorie (40m) an der Konvergenzposition (330) vorzugsweise unabhängig von der Strahlenergie (Ej) des Teilchenstrahls ist.

3. Geladene-Teilchen-Strahl-Therapiesystem nach Anspruch 2, wobei der Winkel der Strahltrajektorie (40e) an der Konvergenzposition (330) von der Strahlenergie (Ej) abhängt und wobei das Strahltransportsystem (30) ein stromabwärts gelegenes Lenkelement (35d) umfasst, das sich an der Konvergenzposition (330) befindet und dazu konfiguriert ist, die Strahltrajektorien in eine gemeinsame Strahltrajektorie unabhängig von der Strahlenergie (Ej) abzulenken.

4. Geladene-Teilchen-Strahl-Therapiesystem nach einem der Ansprüche 1 bis 3, wobei die Biegeeinheit (33) eine oder mehrere Biegeuntereinheiten (33n) umfasst, die entlang der Strahltrajektorie angeordnet sind, wobei jede Biegeuntereinheit (33n) einen Spalt (33g) umfasst, der sich entlang einer gekrümmten Geometrie auf der Biegeebene (Y, Z) von einem Untereinheitseinlass (33ni) zu einem Untereinheitsauslass (33no) erstreckt und die eine erste und eine zweite Magnetspule (33c) separiert, die symmetrisch auf beiden Seiten der Biegeebene positioniert sind, wobei entweder
• die erste und die zweite Magnetspule (33c) einen ersten bzw. zweiten Magnetpol (33p) umgeben, der aus einem ferromagnetischen Material gefertigt ist, oder
• die erste und die zweite Magnetspule (33c) aus einem supraleitenden Material gefertigt sind.

5. Geladene-Teilchen-Strahl-Therapiesystem nach Anspruch 4, wobei der Spalt (33g) wie folgt definiert ist:
• in einer Ebene parallel zu der Biegeebene (Y, Z) definiert der Spalt (33g) eine Biegung, die um die Mediantrajektorie (40m) zentriert ist und zwischen einer nahen Wand (33gc) mit kleinen Krümmungsradius (R1) und einer fernen Wand mit großem Krümmungsradius (R2) (R1 < R2) begrenzt ist, wobei eine Entfernung (hi, ho), die die nahe Wand von der fernen Wand separiert, an dem Biegeeinheitseinlass (33i) größer ist als an dem Biegeeinheitsauslass (33o) des Spalts (33g) ist,
• der Spalt (33g) weist eine Breite (w1, w2), gemessen parallel zu einer X-Achse normal zu der Biegeebene (d. h. X ⊥ Y ⊥ Z), auf, die an der nahen Wand größer als an der fernen Wand ist (w1 > w2).

6. Geladene-Teilchen-Strahl-Therapiesystem nach einem der vorhergehenden Ansprüche, wobei ein Magnetfeld (B33) in dem Spalt (33g) jeder Biegeuntereinheit (33n) einen höchsten Wert in einem Gebiet angrenzend an die ferne Wand aufweist und einen niedrigsten Wert in einem Gebiet angrenzend an die nahe Wand aufweist.

7. Geladene-Teilchen-Strahl-Therapiesystem nach Anspruch 6, wobei der höchste Wert des Magnetfelds (B33) in dem Spalt (33g) jeder Biegeuntereinheit (33n) in folgendem Bereich enthalten ist:
• zwischen 1,5 T und 2,6 T, vorzugsweise zwischen 1,9 T und 2,4 T, falls die erste und zweite Magnetspule (33c) einen ersten und zweiten Magnetpol (33p) umgeben, die aus einem ferromagnetischen Material gefertigt sind, oder
• zwischen 2,5 T und 9 T, vorzugsweise zwischen 3,5 T und 6 T, falls die erste und zweite Magnetspule (33c) supraleitend sind.

8. Geladene-Teilchen-Strahl-Therapiesystem nach einem der Ansprüche 4 bis 7, wobei ein Untereinheitseinlass (33i) und/oder ein Untereinheitsauslass (33o) wenigstens einer Biegeuntereinheit der Biegeeinheit (33) nicht normal zu der Mediantrajektorie (40m) ist.

9. Geladene-Teilchen-Strahl-Therapiesystem nach einem der vorhergehenden Ansprüche, wobei die Teilchen aus Hadronen, vorzugsweise Protonen, Kohlenstoffionen, Heliumionen und Sauerstoffionen ausgewählt sind.

10. Geladene-Teilchen-Strahl-Therapiesystem nach einem der Ansprüche 1 bis 9, wobei das stromaufwärts gelegene Lenkelement (35u) ist entweder
• ein Dipol, der dazu konfiguriert ist, nur die Ablenkfunktion bereitzustellen, oder
• einen Dipol, der zum Bereitstellen einer Ablenkfunktion und einer 1D-Scanfunktion konfiguriert ist, die zum Scannen der Strahltrajektorie in einer Richtung quer zu der Strahltrajektorie konfiguriert ist, um Scannen des Ziels zu ermöglichen,
• ein Oktupol, der dazu konfiguriert ist, die Ablenkfunktion bereitzustellen, und
∘ eine Fokussierungsfunktion, die zum Reduzieren der Größe des Teilchenstrahlquerschnitts in einer oder zwei Richtungen quer zu der Strahltrajektorie konfiguriert ist, und/oder
∘ eine 1D- oder 2D-Scanfunktion, die zum Scannen der Strahltrajektorie in einer oder zwei Richtungen quer zu der Strahltrajektorie konfiguriert ist, um Scannen des Ziels (11) zu ermöglichen.

11. Geladene-Teilchen-Strahl-Therapiesystem nach einem der vorhergehenden Ansprüche, wobei das Strahltransportsystem (30) Folgendes umfasst:
• ein oder mehrere Fokussierungselemente (31) zum Fokussieren des beschleunigten Strahls entlang der Mediantrajektorie (40m) in Querschnittsebenen (X, Y), die normal zu der Mediantrajektorie (40m) sind, und
• vorzugsweise ist ein Scanner zum Ablenken der Strahltrajektorien um einen Winkel, der zwischen -15° und +15° grad liegt, in Richtungen normal zu der Mediantrajektorie (40m), und der zum Steuern der Strahltrajektorien zum Scannen über das Ziel (11) konfiguriert ist.

## Revendications

1. Système de thérapie par faisceau de particules chargées comprenant :
• une unité de patient (10) permettant de soutenir un patient dans une position donnée,
• une unité d'accélérateur (20) configurée pour accélérer des particules chargées et délivrer hors d'une sortie (20o) un faisceau de particules de particules chargées accélérées d'énergie comprise entre 40 et 450 MeV/u (= MeV par nucléon),
• un système de transport de faisceau (30) configuré pour guider le faisceau de particules d'énergie (Ej, j = 1 à 3) variant entre 40 et 450 MeV/u, le long de trajectoires de faisceaux correspondantes (40e) centrées sur une trajectoire médiane (40m) partant de la sortie (20o) de l'unité d'accélérateur (20) jusqu'à une cible (11) située au sein de l'unité de patient (10), où le système de transport de faisceau (30) comprend
• une unité de cintrage (33) comportant un intervalle (33g) entre les pôles (33p) d'un dipôle à champ fixe configuré pour recevoir et cintrer la trajectoire médiane (40m) dans un plan de cintrage (y, Z) d'un angle compris entre 20 et 270°, de préférence entre 40 et 140° dans le plan de cintrage, entre une entrée (33i) de l'unité de cintrage et une sortie (33o) de l'unité de cintrage, la trajectoire médiane (40m) étant comprise dans le plan de cintrage (Y, Z), avec Y ⊥ Z,
**caractérisé en ce que**
• le système de transport de faisceau (30) est un portique configuré pour faire tourner le plan de cintrage (Y, Z) autour d'un axe de rotation passant par la cible (11),
• le système de transport de faisceau (30) comprend en outre un déflecteur amont (35u) situé en amont de l'unité de cintrage (33) et qui est configuré pour dévier les trajectoires de faisceau (40e) en éloignement de la trajectoire médiane (40m) d'un angle (θ35) compris entre -15 et +15° dans le plan de cintrage (Y, Z), et **en ce que**
• le déflecteur amont (35u) est configuré pour fournir une fonction de déviation, obtenue par la création d'un champ magnétique uniforme (B35) dans le déflecteur amont (35u), où une grandeur du champ magnétique uniforme (B35) peut être contrôlée pour faire varier une position d'entrée (Yij, avec j = 1 à 3)) et un angle d'entrée (θ33i) de la trajectoire du faisceau à l'entrée (33i) de l'unité de cintrage en fonction d'énergie (Ej, avec j = 1 à 3) du faisceau de particules.

2. Système de thérapie par faisceau de particules chargées selon la revendication 1, comprenant un processeur configuré pour contrôler l'amplitude du champ magnétique uniforme (B35) du déflecteur amont (35u) en fonction de l'énergie du faisceau (Ej), pour contrôler la position (Yij) et l'angle d'entrée (θ33i) de la trajectoire du faisceau à l'entrée (33i) de l'unité de cintrage de manière à entraîner la trajectoire du faisceau (40e) le long d'une trajectoire courbe de rayon de cintrage correspondant à l'énergie du faisceau (Ej), et passant par une position convergente (330) en aval de la sortie (33o) de l'unité de cintrage, un angle de la trajectoire (40e) du faisceau par rapport à la trajectoire médiane (40m) au niveau de la position convergente (330) étant de préférence indépendant de l'énergie (Ej) du faisceau de particules.

3. Système de thérapie par faisceau de particules chargées selon la revendication 2, dans lequel l'angle de la trajectoire de faisceau (40e) à la position convergente (330) dépend de l'énergie de faisceau (Ej) et dans lequel le système de transport de faisceau (30) comprend un déflecteur aval (35d) situé à la position convergente (330) et configuré pour dévier les trajectoires de faisceau selon une trajectoire de faisceau commune indépendante de l'énergie de faisceau (Ej).

4. Système de thérapie par faisceau de particules chargées selon l'une quelconque des revendications 1 à 3, dans lequel l'unité de cintrage (33) comprend une ou plusieurs sous-unités de cintrage (33n) disposées le long de la trajectoire du faisceau, dans lequel chaque sous-unité de cintrage (33n) comprend un intervalle (33g) s'étendant le long d'une géométrie courbe sur le plan de cintrage (Y, Z) depuis une entrée de sous-unité (33ni) jusqu'à une sortie de sous-unité (33no) et séparant des première et deuxième bobines magnétiques (33c) positionnées symétriquement de chaque côté du plan de cintrage, où,
• soit les première et deuxième bobines magnétiques (33c) entourent respectivement des premier et deuxième pôles magnétiques (33p) constitués de matériau ferromagnétique,
• soit les première et deuxième bobines magnétiques (33c) sont constituées d'un matériau supraconducteur.

5. Système de thérapie par faisceau de particules chargées selon la revendication 4, dans lequel l'intervalle (33g) est défini comme suit
• dans un plan parallèle au plan de cintrage (Y, Z), l'intervalle (33g) définit un coude centré sur la trajectoire médiane (40m) et délimité entre une paroi rapprochée (33gc) de faible rayon de courbure (R1) et une paroi éloignée de grand rayon de courbure (R2) (R1 < R2), où une distance (hi, ho) séparant la paroi proche de la paroi éloignée est plus grande au niveau de l'entrée (33i) de l'unité de cintrage qu'au niveau de la sortie (33o) de l'intervalle (33g),
• l'intervalle (33g) a une largeur (w1, w2) mesurée parallèlement à un axe X normal au plan de cintrage (c.-à-d. X ⊥ y ⊥ Z), plus grande au niveau de la paroi proche qu'au niveau de la paroi éloignée (w1 > w2).

6. Système de thérapie par faisceau de particules chargées selon l'une quelconque des revendications précédentes, dans lequel un champ magnétique (B33) dans l'intervalle (33g) de chaque sous-unité de cintrage (33n) a la valeur la plus élevée dans une région adjacente à la paroi éloignée, et a la valeur la plus faible dans une région adjacente à la paroi proche.

7. Système de thérapie par faisceau de particules chargées selon la revendication 6, dans lequel la valeur la plus élevée du champ magnétique (B33) dans l'intervalle (33g) de chaque sous-unité de cintrage (33n) est comprise,
• entre 1,5 T et 2,6 T, de préférence entre 1,9 T et 2,4 T, si les première et deuxième bobines magnétiques (33c) entourent les premier et deuxième pôles magnétiques (33p) constitués de matériau ferromagnétique, ou
• entre 2,5 T et 9 T, de préférence entre 3,5 T et 6 T si les première et deuxième bobines magnétiques (33c) sont supraconductrices.

8. Système de thérapie par faisceau de particules chargées selon l'une quelconque des revendications 4 à 7, dans lequel une entrée de sous-unité (33i) et/ou une sortie de sous-unité (33o) d'au moins une sous-unité de cintrage de l'unité de cintrage (33) n'est pas normale à la trajectoire médiane (40m).

9. Système de thérapie par faisceau de particules chargées selon l'une quelconque des revendications précédentes, dans lequel les particules sont choisies parmi des hadrons, de préférence des protons, des ions carbone, des ions hélium et des ions oxygène.

10. Système de thérapie par faisceau de particules chargées selon l'une quelconque des revendications 1 à 9, dans lequel le déflecteur amont (35u) est
• soit un dipôle configuré pour assurer uniquement la fonction de déviation,
• soit un dipôle configuré pour fournir une fonction de déviation et une fonction de balayage 1D configurée pour balayer la trajectoire de faisceau dans une direction transversale à la trajectoire de faisceau pour permettre un balayage de la cible,
• soit un octupole configuré pour fournir la fonction de déviation et,
∘ une fonction de focalisation configurée pour réduire la taille de la section transversale du faisceau de particules dans une ou deux directions transversales à la trajectoire du faisceau, et/ou
∘ une fonction de balayage 1D ou 2D configurée pour balayer la trajectoire de faisceau dans une ou deux directions transversales à la trajectoire de faisceau afin de permettre le balayage de la cible (11).

11. Système de thérapie par faisceau de particules chargées selon l'une quelconque des revendications précédentes, dans lequel le système de transport de faisceau (30) comprend :
• un ou plusieurs éléments de focalisation (31) pour focaliser le faisceau accéléré le long de la trajectoire médiane (40m) dans des plans de coupe transversale (X, Y) perpendiculaires à la trajectoire médiane (40m), et
• de préférence, un scanner pour dévier les trajectoires de faisceau d'un angle compris entre -15° et +15° dans des directions perpendiculaires à la trajectoire médiane (40m) et configuré pour contrôler les trajectoires de faisceau pour balayer la cible (11).
